(19) Europäisches Patentamt
European Patent Office
Office européen des brevets

(11) Veröffentlichungsnummer: **0 135 181**
**B1**

(12) # EUROPÄISCHE PATENTSCHRIFT

(45) Veröffentlichungstag der Patentschrift: **31.01.90**

(51) Int. Cl.⁴: **C 07 K 5/06**, C 07 K 1/08, A 61 K 37/02

(21) Anmeldenummer: **84110677.6**

(22) Anmeldetag: **07.09.84**

(54) **Verfahren zur Herstellung N-alkylierter Dipeptide und deren Estern.**

(30) Priorität: **16.09.83 DE 3333455**

(43) Veröffentlichungstag der Anmeldung: **27.03.85 Patentblatt 85/13**

(45) Bekanntmachung des Hinweises auf die Patenterteilung: **31.01.90 Patentblatt 90/5**

(84) Benannte Vertragsstaaten: **AT BE CH DE FR GB IT LI LU NL SE**

(56) Entgegenhaltungen:
**EP-A- 0 049 605**
**EP-A- 0 079 022**
**EP-A- 0 088 342**
**EP-A- 0 089 637**
**DE-A- 2 901 843**
**DE-A- 3 315 464**

**Arzneimittelforschung, 34 (II), Nr. 10b (1984), Seiten 1399-1401**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Rotenhofstrasse 31, D-6234 Hattersheim am Main (DE)**
Erfinder: **Wissmann, Hans, Dr., Falkenstrasse 12, D-6232 Bad Soden am Taunus (DE)**
Erfinder: **Teetz, Volker, Dr., An der Tann 20, D-6238 Hofheim am Taunus (DE)**

**EP 0 135 181 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Aus der EP-A-0 049 605 ist ein Verfahren zur Herstellung von N-Acylderivaten der 1,2,3,4-Tetrahydroisochinolin-3-carbonsäure durch Kupplung eines 1,2,3,4-Tetrahydroisochinolin-3-carboxylats mit einer N-substituierten Aminosäure in Gegenwart von Dehydratisierungsmitteln und Reaktivester bildender Reagentien bekannt.

Die Erfindung betrifft ein Verfahren zur Herstellung von Verbindungen der Formel I

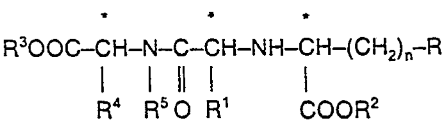 (I)

in welcher
n = 1 oder 2 ist,
R = Wasserstoff,
einen gegebenenfalls substituierten aliphatischen Rest mit 1 bis 8 C-Atomen,
einen gegebenenfalls substituierten alicyclischen Rest mit 3–9 C-Atomen,
einen gegebenenfalls substituierten aromatischen Rest mit 6–12 C-Atomen,
einen gegebenenfalls substituierten araliphatischen Rest mit 7–14 C-Atomen,
einen gegebenenfalls substituierten alicyclischaliphatischen Rest mit 7–14 C-Atomen,
einen Rest $OR^a$ oder $SR^a$, worin
$R^a$ für einen gegebenenfalls substituierten aliphatischen Rest mit 1–4 C-Atomen, für einen gegebenenfalls substituierten aromatischen Rest mit 6–12 C-Atomen oder einen gegebenenfalls substituierten heteroaromatischen Rest mit 5–12 Ringatomen steht,
$R^1$ Wasserstoff,
einen gegebenenfalls substituierten aliphatischen Rest mit 1 bis 6 C-Atomen,
einen gegebenenfalls substituierten alicyclischen Rest mit 3–9 C-Atomen,
einen gegebenenfalls substituierten alicyclischaliphatischen Rest mit 4–13 C-Atomen,
einen gegebenenfalls substituierten aromatischen Rest mit 6–12 C-Atomen,
einen gegebenenfalls substituierten araliphatischen Rest mit 7–16 C-Atomen,
einen gegebenenfalls substituierten heteroaromatischen Rest mit 5–12 Ringatomen oder
die erforderlichenfalls geschützte Seitenkette einer natürlich vorkommenden α-Aminosäure bedeuten,
$R^2$ und $R^3$ gleich oder verschieden sind und Wasserstoff, einen gegebenenfalls substituierten aliphatischen Rest mit 1–6 C-Atomen,
einen gegebenenfalls substituierten alicyclischen Rest mit 3–9 C-Atomen,
einen gegebenenfalls substituierten aromatischen Rest mit 6–12 C-Atomen,
einen gegebenenfalls substituierten araliphatischen Rest mit 7–16 C-Atomen bedeuten und
$R^4$ und $R^5$ zusammen mit den sie tragenden Atomen ein heterocyclisches, mono-, bi-, oder tricyclisches Ringsystem mit 5 bis 15 C-Atomen bilden, dadurch gekennzeichnet, daß man Verbindungen der Formel II

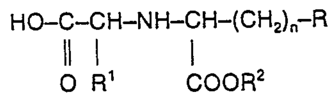 (II),

in welcher n, R, $R^1$ und $R^2$ die oben definierten Bedeutungen haben, in Gegenwart von Alkan-Phosphonsäureanhydriden, mit Verbindungen der Formel III

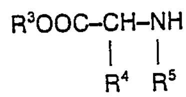 (III)

in welcher $R^3$, $R^4$ und $R^5$ die oben definierten Bedeutungen haben, umsetzt, gegebenenfalls zum Schutz anderer funktioneller Gruppen eingeführte Reste abspaltet und gegebenenfalls freie Carboxygruppen in an sich bekannter Weise verestert.

Als solche Ringsysteme kommen insbesondere jene aus der folgenden Gruppe in Betracht:

Pyrrolidin (A); Piperidin (B); Tetrahydroisochinolin (C); Decahydroisochinolin (D); Octahydroindol (E); Octahydrocyclopenta[b]pyrrol (F); 2-Azabicyclo[2.2.2]octan (G); 2-Azabicyclo[2.2.1]heptan (H); 2-Azaspiro[4.5]decan (I); 2-Azaspiro[4.4]nonan (J); Spiro[(bicyclo[2.2.1]heptan)-2,3-pyrrolidin] (K); Spiro[(bicyclo[2.2.2]octan)-2,3-pyrrolidin] (L); 2-Azatricyclo[4,3,0,$1^{6,9}$]decan (M); Decahydrocyclohepta[b]pyrrol (N); Octahydroisoindol (O); Octahydrocyclopenta[c]pyrrol (P); 2,3,3a,4,5,7a-Hexahydroindol (Q); Tetrahydrothiazol (R); 2-Azabicyclo[3.1.0]hexan (S); die alle gegebenenfalls substituiert sein können. Bevorzugt sind jedoch die unsubstituierten Systeme.

Bei den Verbindungen, die mehrere chirale Atome besitzen, kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf.

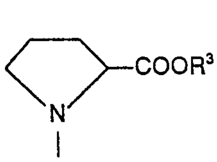
A

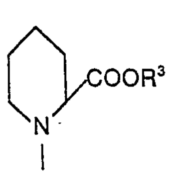
B

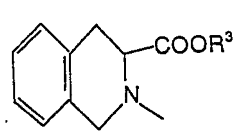
C

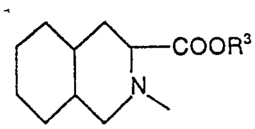
D

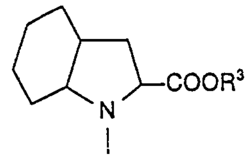

E

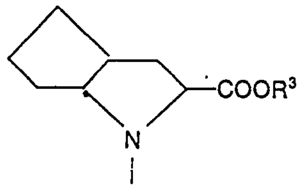

F

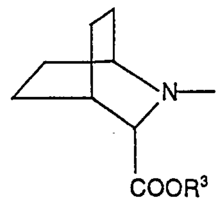

G

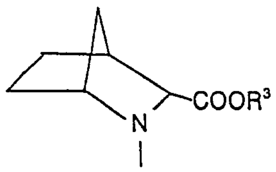

H

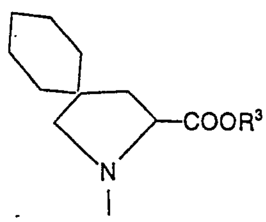

I

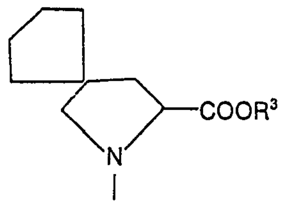

J

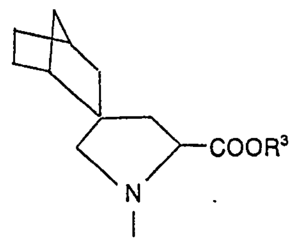

K

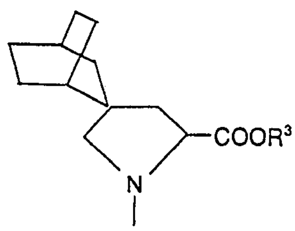

L

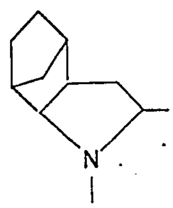

M

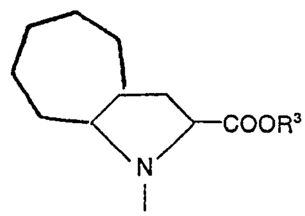

N

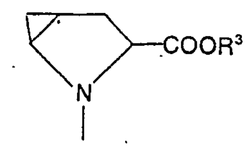

S

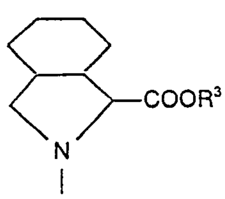

O

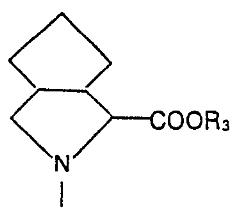

P

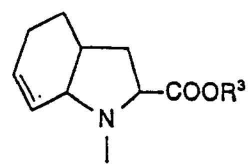

Q

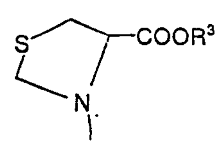

R

Zur Herstellung von Carbonsäureamid- und Peptidbindungen ist eine große Anzahl von Verfahren bekannt (siehe z.B. Houben-Weyl, Methoden der organischen Chemie, Bd. XV, Teil II, S. 1–364. Ferner Angew. Chemie 92, 129 (1980)). Alle diese Verfahren zielen mit unterschiedlichem Erfolg darauf, die für die Synthese von Peptiden notwendigen Kriterien der Racemisierungsfreiheit, der einfachen und schonenden Durchführbarkeit bei hohen Ausbeuten und mit leicht zugänglichen, möglichst ungefährlichen Ausgangsmaterialien sicherzustellen.

Aus dem US-Patent 4 331 592 ist ein Verfahren zur Herstellung von Carbonsäureamidgruppen enthaltenden Verbindungen durch Umsetzung von COOH-Gruppen enthaltenden Verbindungen mit Verbindungen, die eine freie $NH_2$-Gruppe enthalten, in Gegenwart von Anhydriden der Alkanphosphonsäuren bekannt.

Die Ausbeuten bei den bisher bekannten Methoden zur Herstellung von Verbindungen der Formel I ausgehend von Verbindungen der Formeln II und III (z.B. der HOBt-DCCI-Methode mit DMF oder Acetonitril als Lösungsmittel) liegen bei 50–75%. Im Falle des DCCI bereitet die restlose Entfernung des entstehenden Dicyclohexylharnstoffs Schwierigkeiten, außerdem sind schwere Allergien gegen Carbodiimide bekannt. Andere Reagentien, z.B. andere Anhydride von Phosphorsäuren sind prinzipiell geeignet, das HOBt-Verfahren zu ersetzen, jedoch gilt es zu reaktive Reagentien zu vermeiden, um Nebenreaktionen (beispielsweise mit der ungeschützten sekundären Aminogruppe in der Verbindung der Formel II) zu verhindern.

Das vorliegende Verfahren stellt einen neuen Weg dar, die genannten Bedingungen für eine wirtschaftliche Synthese von Verbindungen der Formel I zu optimieren. Mit Hilfe des erfindungsgemäßen Verfahrens lassen sich Verbindungen der Formel II mit solchen der Formel III unter milden Bedingungen in guter Ausbeute zu Verbindungen der Formel I umsetzen. Es ist überraschend, daß es dabei nicht zu Nebenreaktionen an der ungeschützten sekundären Aminogruppe in den Verbindungen der Formel II bzw. dem Endprodukt kommt.

Die zum Schutz der funktionellen Gruppen eingeführten Reste können anschließend in üblicher Weise abgespalten werden.

Nach dem erfindungsgemäßen Verfahren geeignet sind Anhydride der grad- oder verzweigtkettigen, gegebenenfalls cyclischen Alkanphosphonsäuren mit Kettenlängen von 1–8 Kohlenstoffatomen, bevorzugt bis zu 4 Kohlenstoffatomen.

Die erfindungsgemäß verwendeten Phosphonsäureanhydride sind bei Raumtemperatur stabil. In den meisten nicht wäßrigen Lösungsmitteln, insbesondere in Lipidlösungsmitteln wie Chloroform oder Methylenchlorid, aber auch in polaren Lösungsmitteln wie DMF und DMA sind sie leicht löslich.

Als Anhydride der Alkanphosphonsäure seien beispielsweise genannt:
Methanphosphonsäureanhydrid, Äthanphosphonsäureanhydrid, n-Propanphosphonsäureanhydrid, n-Butanphosphonsäureanhydrid, insbesondere n-Propanphosphonsäureanhydrid.

Die Herstellung der Alkanphosphonsäureanhydride kann in an sich bekannter Weise erfolgen, wie z.B. in Houben-Weyl, Methoden der Organischen Chemie, G. Thieme Verl., Stuttgart 1963, Bd. XII/1, S. 612 formuliert.

Eine bevorzugte Ausführungsform ist dadurch gekennzeichnet, daß die Verbindungen der Formel I hergestellt werden, in der
n = 1 oder 2 ist,
R Wasserstoff,
Alkyl mit 1–8 C-Atomen,
Alkenyl mit 2–6 C-Atomen,
Cycloalkyl mit 3–9 C-Atomen,
Aryl mit 6–12 C-Atomen,
das durch ($C_1$–$C_4$)-Alkyl, ($C_1$–$C_4$)-Alkoxy, Hydroxy, Halogen, Nitro, Amino, Aminomethyl, ($C_1$–$C_4$)-Alkylamino, Di-($C_1$–$C_4$)-alkylamino, ($C_1$–$C_4$)-Acylamino, vorzugsweise ($C_1$–$C_4$)-Alkanoylamino, Methylendioxy, Carboxy, Cyano und/oder Sulfamoyl mono-, di- oder trisubstituiert sein kann,
Alkoxy mit 1–4 C-Atomen,
Aryloxy mit 6–12 C-Atomen,
das wie oben bei Aryl beschrieben substituiert sein kann,
mono bzw. bicyclisches Heteroaryloxy mit 5–7 bzw. 8–10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$–$C_4$)-alkyl,
($C_1$–$C_4$)-Alkanoylamino-($C_1$–$C_4$)-alkyl,
($C_7$–$C_{13}$)-Aroylamino-($C_1$–$C_4$)-alkyl,
($C_1$–$C_4$)-Alkoxy-carbonylamino-($C_1$–$C_4$)-alkyl,
($C_6$–$C_{12}$)-Aryl-($C_1$–$C_4$)-alkoxycarbonylamino-($C_1$–$C_4$)-alkyl,
($C_6$–$C_{12}$)-Aryl-($C_1$–$C_4$)-alkylamino-($C_1$–$C_4$)-alkyl,
($C_1$–$C_4$)-Alkylamino-($C_1$–$C_4$)-alkyl,
Di-($C_1$–$C_4$)-alkylamino-($C_1$–$C_4$)-alkyl,
Guanidino-($C_1$–$C_4$)-alkyl,
Imidazolyl, Indolyl,
($C_1$–$C_4$)-Alkylthio,
($C_1$–$C_4$)-Alkylthio-($C_1$–$C_4$)-alkyl,
($C_6$–$C_{12}$)-Arylthio-($C_1$–$C_4$)-alkyl,
das im Arylteil wie oben bei Aryl beschrieben, substituiert sein kann,
($C_6$–$C_{12}$)-Aryl-($C_1$–$C_4$)-alkylthio,
das im Arylteil wie oben bei Aryl beschrieben, substituiert sein kann,
Carboxy-($C_1$–$C_4$)-alkyl,
Carboxy, Carbamoyl,
Carbamoyl-($C_1$–$C_4$)-alkyl,
($C_1$–$C_4$)-Alkoxy-carbonyl-($C_1$–$C_4$)-alkyl,
($C_6$–$C_{12}$)-Aryloxy-($C_1$–$C_4$)-alkyl,
das im Arylteil wie oben bei Aryl beschrieben, substituiert sein kann oder
($C_6$–$C_{12}$)-Aryl-($C_1$–$C_4$)-alkoxy,
das im Arylteil wie oben bei Aryl beschrieben, substituiert sein kann.
$R^1$ Wasserstoff,
Alkyl mit 1–6 C-Atomen,
Alkenyl mit 2–6 C-Atomen,
Alkinyl mit 2–6 C-Atomen,
Cycloalkyl mit 3–9 C-Atomen,
Cycloalkenyl mit 5–9 C-Atomen,
($C_3$–$C_9$)-Cycloalkyl-($C_1$–$C_4$)-alkyl,
($C_5$–$C_9$)-Cycloalkenyl-($C_1$–$C_4$)-alkyl,
gegebenenfalls teilhydriertes Aryl mit 6–12 C-Atomen, das wie oben bei R beschrieben, substituiert sein kann,
($C_6$–$C_{12}$)-Aryl-($C_1$–$C_4$)-alkyl oder ($C_7$–$C_{13}$)-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5–7 bzw. 8–10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlich vorkommenden α-Aminosäure $R^1$–$CH(NH_2)$–$COOH$ bedeuten,
$R^2$ und $R^3$ gleich oder verschieden sind und
Wasserstoff,
Alkyl mit 1–6 C-Atomen,
Alkenyl mit 2–6 C-Atomen,
Di-($C_1$–$C_4$)-alkylamino-($C_1$–$C_4$)-alkyl,
($C_1$–$C_5$)-Alkanoyloxy-($C_1$–$C_4$)-alkyl,
($C_1$–$C_6$)-Alkoxy-carbonyloxy-($C_1$–$C_4$)-alkyl,
($C_7$–$C_{13}$)-Aroyloxy-($C_1$–$C_4$)-alkyl,
($C_6$–$C_{12}$)-Aryloxycarbonyloxy-($C_1$–$C_4$)-alkyl,
Aryl mit 6–12 C-Atomen,
($C_1$–$C_{12}$)-Aryl-($C_1$–$C_4$)-alkyl,
($C_3$–$C_9$)-Cycloalkyl oder
($C_3$–$C_9$)-Cycloalkyl-($C_1$–$C_4$)-alkyl
bedeuten und

$R^4$ und $R^5$ die oben angegebene Bedeutung haben, wobei während der Umsetzung gegebenenfalls in den Resten R bis $R^5$ vorhandene freie Amino-, Alkylamino-, Hydroxy-, Carboxy-, Mercapto- und/oder Guanidinofunktionen in an sich bekannter Weise blockiert werden (vgl. z.B. Kontakte Merck 3/79, S. 14 ff. und 1/80, S. 23 ff.).

Besonders bevorzugt ist eine Ausführungsform, die dadurch gekennzeichnet ist, daß Verbindungen der Formel I hergestellt werden, in der

n = 1 oder 2 ist,

R ($C_1$ bis $C_6$)-Alkyl, ($C_2$ bis $C_6$)-Alkenyl, ($C_3$ bis $C_9$)-Cycloalkyl, Amino-($C_1$–$C_4$)-alkyl, ($C_2$–$C_5$)-Acylamino-($C_1$–$C_4$)-alkyl, ($C_7$–$C_{13}$)-Aroylamino-($C_1$–$C_4$)-alkyl, ($C_1$–$C_4$)-Alkoxycarbonylamino-($C_1$–$C_4$)-alkyl, ($C_6$ bis $C_{12}$)-Aryl-($C_1$–$C_4$)-alkoxycarbonylamino-($C_1$–$C_4$)-alkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkyl-amino und/oder Methylendioxy mono-, di- oder trisubstituiert sein kann, oder 3-Indolyl, insbesondere Methyl, Ethyl, Cyclohexyl, tert. Butoxycarbonylamino-($C_1$–$C_4$)-alkyl, Benzoyloxycarbonylamino-($C_1$–$C_4$)-alkyl oder Phenyl, das durch Phenyl, ($C_1$ bis $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkyl-amino, Nitro und/oder Methylendioxy mono- oder disubstituiert oder im Falle von Methoxy, trisubstituiert sein kann, bedeutet.

$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$–$C_{12}$)-Aryl-($C_1$ bis $C_4$)-alkyl oder ($C_7$–$C_{13}$)-Aroyl-($C_1$–$C_2$)-alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$ bis $C_3$)-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl, insbesondere aber Wasserstoff, ($C_1$ bis $C_4$)-Alkyl oder Benzyl bedeuten und

$R^4$ und $R^5$ die oben angegebene Bedeutung haben, wobei andere funktionelle Gruppen während der Umsetzung, wie oben beschrieben, blockiert werden.

Insbesondere bevorzugt ist beispielsweise ein Verfahren, das zu Verbindungen der Formel I führt, in welcher

n = 2 ist, R = Phenyl, $R^1$ = Methyl, $R^2$ und $R^3$ gleiche oder verschiedene ($C_1$ bis $C_6$)-Alkyl-Reste oder ($C_7$ bis $C_{10}$)-Aralkyl-Reste wie Benzyl oder Nitrobenzyl) bedeuten und

$R^4$ und $R^5$ zusammen für ein Rest der Formel steht,

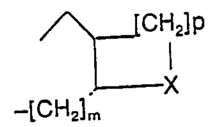

worin

m = 0 oder 1, p = 0,1 oder 2 und X = $-CH_2-$, $-CH_2-CH_2-$ oder $-CH=CH-$ bedeuten, wobei ein mit X gebildeter 6-Ring auch ein Benzolring sein kann.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$–$C_6$)-Alkanoyl bevorzugt. Als O-Schutzgruppen für Tyrosin kommen bevorzugt ($C_1$–$C_6$)-Alkyl, insbesondere Methyl oder Ethyl in Frage.

Besonders vorteilhaft können die folgenden Verbindungen nach dem erfindungsgemäßen Verfahren erhalten werden.

N-(1-S-Carbethoxy-3-phenyl-propyl)-S-alanyl-S-prolin-benzylester

N-(1-R-Carbethoxy-3-phenyl-propyl)-S-alanyl-S-prolin-benzylester

N-(1-R,S-Carbethoxy-3-phenyl-propyl)-S-alanyl-S-prolin-benzylester

N-(1-S-Carbethoxy-3-cyclohexyl-propyl)-S-alanyl-S-prolin-benzylester

N-(1-R-Carbethoxy-3-cyclohexyl-propyl)-S-alanyl-S-prolin-benzylester
N-(1-R,S-Carbethoxy-3-cyclohexyl-propyl)-S-alanyl-S-prolin-benzylester
N-(1-S-Carbethoxy-3-phenyl-propyl)-Nε-benzyloxycarbonyl-S-lysyl-S-prolin-tert. butylester
N-(1-S-Carbethoxy-3-phenyl-propyl)-S-tyrosyl-S-prolin-benzylester
N-(1-S-Carbethoxy-3-phenyl-propyl)-O-methyl-S-tyrosyl-S-prolin-benzylester
N-(1-S-Carbethoxy-3-phenyl-propyl)-O-ethyl-S-tyrosyl-S-prolin-benzylester
N-(1-S-Carbethoxy-3-phenyl-propyl)-S-alanyl-S-pipecolsäure-benzylester
N-(1-S-Carbethoxy-3-cyclohexyl-propyl)-S-alanyl-S-pipecolsäurebenzylester
N-(1-S-Carbethoxy-3-phenyl-propyl)-S-alanyl-S-1,2,3,4-tetrahydroisochinolin-3-carbonsäure-tert. butylester
N-(1-S-Carbethoxy-3-cyclohexyl-propyl)-S-alanyl-S-1,2,3,4-tetrahydroisochinolin-3-carbonsäure-tert. butylester
N-(1-S-Carbethoxy-3-phenyl-propyl)-Nε-benzylcarbonyl-S-lysyl-S-1,2,3,4-tetrahydroisochinolin-3-carbonsäure-tert. butylester
N-(1-S-Carbethoxy-3-phenyl-propyl)-O-ethyl-S-tyrosyl-S-1,2,3,4-tetrahydroisochinolin-3-carbonsäure-tert. butylester
N-(1-S-Carbethoxy-3-phenyl-propyl)-S-alanyl-3S-decahydroisochinolin-3-carbonsäure-benzylester
N-(1-S-Carbethoxy-3-phenyl-propyl)-S-alanyl-(2S,3aS,7aS)-octahydroindol-2-carbonsäure-benzylester
N-(1-S-Carbethoxy-3-cyclohexyl-propyl)-S-alanyl-(2S, 3aS, 7aS)-octahydroindol-2-carbonsäure-benzylester
N-(1-S-Carbethoxy-3-phenyl-propyl)-Nε-benzyloxycarbonyl-S-lysyl-(2S,3aS,7aS)-octahydroindol-2-carbonsäure-tert. butylester
N-(1-S-Carbethoxy-3-cyclohexyl-propyl)-Nε-benzyloxycarbonyl-S-lysyl-(2S,3aS,7aS)-octahydroindol-2-carbonsäure-tert. butylester
N-(1-S-Carbethoxy-3-phenyl-propyl)-O-methyl-S-tyrosyl-(2S,3aS,7aS)-octahydroindol-2-carbonsäure-benzylester
N-(1-S-Carbethoxy-3-phenyl-propyl)-O-ethyl-S-tyrosyl-(2S,3aS,7aS)-octahydroindol-2-carbonsäure-benzylester
N-(1-S-Carbethoxy-4,4-dimethylphenyl)-O-alanyl-(2S,3aS,7aS)-octahydroindol-2-carbonsäure-benzylester
N-[1-S-Carbethoxy-3-(4-fluorphenyl)-propyl]-S-alanyl-(2S,3aS,7aS)-octahydroindol-2-carbonsäure-benzylester
N-[1-S-Carbethoxy-3-(4-methoxyphenyl)-propyl]-S-alanyl-(2S,3aS,7aS)-octahydroindol-2-carbonsäure-benzylester
N-[1-S-Carbethoxy-3-(3,4-dimethoxyphenyl)-propyl]-S-alanyl-(2S,3aS,7aS)-octahydroindol-2-carbonsäure-benzylester
N-(1-S-Carbethoxy-3-cyclopentylpropyl)-S-alanyl-(2S,3aS,7aS)-octahydroindol-2-carbonsäure-benzylester
N-(1-S-Carbethoxy-3-phenyl-propyl)-S-alanyl-(2S,3aR,7aS)-octahydroindol-2-carbonsäure-benzylester
N-(1-S-Carbethoxy-3-cyclohexyl-propyl)-S-alanyl-(2S,3aR,7aS)-octahydroindol-2-carbonsäure-benzylester
N-(1-S-Carbethoxy-3-phenyl-propyl)-Nε-benzyloxycarbonyl-S-lysyl-(2S,3aR,7aS)-octahydroindol-2-carbonsäure-tert. butylester
N-(1-S-Carbethoxy-3-cyclohexyl-propyl)-Nε-benzyloxy-carbonyl-S-lysyl-(2S,3aR,7aS)-octahydroindol-2-carbonsäure-benzylester
N-(1-S-Carbethoxy-3-phenyl-propyl)-O-ethyl-S-tyrosyl-(2S,3aR,7aS)-octahydroindol-2-carbonsäure-benzylester
N-(1-S-Carbethoxy-3-phenyl-propyl)-S-alanyl-(2S,3aR,7aR)-octahydroindol-2-carbonsäure-benzylester
N-(1-S-Carbethoxy-3-phenyl-propyl)-Nε-benzyloxycarbonyl-S-lysyl-(2S,3aR,7aR)-octahydroindol-2-carbonsäure-tert. butylester
N-(1-S-Carbethoxy-3-cyclohexyl-propyl)-S-alanyl-(2S,3aR,7aR)-octahydroindol-2-carbonsäure-benzylester
N-(1-S-Carbethoxy-3-cyclohexyl-propyl)-O-ethyl-S-tyrosyl-(2S,3aR,7aR)-octahydroindol-2-carbonsäure-benzylester
N-(1-S-Carbethoxy-3-phenyl-propyl)-S-alanyl-(2S,3aS,7aR)-octahydroindol-2-carbonsäure-benzylester
N-(1-S-Carbethoxy-3-phenyl-propyl)-O-ethyl-S-tyrosyl-(2S,3aS,7aS)-octahydroindol-2-carbonsäure-benzylester
N-(1-S-Carbethoxy-4,4-dimethylphenyl)-O-alanyl-(2S,3aS,7aS)-octahydroindol-2-carbonsäure-benzylester
N-[1-S-Carbethoxy-3-(4-fluorphenyl)-propyl]-S-alanyl-(2S,3aS,7aS)-octahydroindol-2-carbonsäure-benzylester
N-[1-S-Carbethoxy-3-(4-methoxyphenyl)-propyl]-S-alanyl-(2S,3aS,7aS)-octahydroindol-2-carbonsäure-benzylester
N-[1-S-Carbethoxy-3-(3,4-dimethoxyphenyl)-propyl]-S-alanyl-(2S,3aS,7aS)-octahydroindol-2-carbonsäure-benzylester
N-(1-S-Carbethoxy-3-cyclopentylpropyl)-S-alanyl-(2S,3aS,7aS)-octahydroindol-2-carbonsäure-benzylester
N-(1-S-Carbethoxy-3-phenyl-propyl)-Nε-benzyloxycarbonyl-S-lysyl-(2S,3aR,6aS)-octahydrocyclopenta[b]pyrrol-2-carbonsäure-tert. butylester
N-(1-S-Carbethoxy-3-cyclohexylpropyl)-Nε-benzyloxycarbonyl-S-lysyl-(2S,3aR,6aS)-octahydrocyclopenta[b]pyrrol-2-carbonsäure-tert. butylester
N-(1-S-Carbethoxy-3-phenyl-propyl)-O-ethyl-S-tyrosyl-(2S,3aR,6aS)-octahydrocyclopenta[b]-pyrrol-2-carbonsäure-benzylester
N-(1-S-Carbethoxy-3-cyclopentyl-propyl)-S-alanyl-2-(2S,3aR,6aS)-octahydrocyclopenta[b]-pyrrol-2-carbonsäure-benzylester
N-(1-S-Carbethoxy-3-phenyl-propyl)-S-alanyl-S-2-azabicyclo[2.2.2]-octan-3-carbonsäure-benzylester

N-(1-S-Carbethoxy-3-phenyl-propyl)-O-ethyl-S-tyrosyl-S-2-azabicyclo[2.2.2]-octan-3-carbonsäurebenzylester
N-(1-S-Carbethoxy-3-cyclohexyl-propyl)-S-alanyl-S-2-azabicyclo[2.2.2]-octan-3-carbonsäure-benzylester
N-(1-S-Carbethoxy-3-cyclohexyl-propyl)-Nε-benzyloxy-carbonyl-S-2-azabicyclo[2.2.2]-octan-3-carbonsäure-tert. butylester
N-(1-S-Carbethoxy-3-phenyl-propyl)-Nε-benzyloxycarbonyl-S-lysyl-S-2-azabicyclo[2.2.2]-octan-3-carbonsäure-tert. butylester
N-(1-S-Carbethoxy-3-cyclopentylpropyl)-S-alanyl-S-2-azabicyclo[2.2.2]-octan-3-carbonsäure-benzylester

N-(1-S-Carbethoxy-3-phenyl-propyl)-S-alanyl-3S-exo-2-azabicyclo[2.2.1]-heptan-3-carbonsäure-benzylester
N-(1-S-Carbethoxy-3-cyclohexylpropyl)-S-alanyl-3S-exo-2-azabicyclo[2.2.1]-heptan-3-carbonsäure-benzylester
N-(1-S-Carbethoxy-3-phenyl-propyl)-Nε-benzyloxy-carbonyl-S-lysyl-3S-exo-2-azabicyclo-[2.2.1]-heptan-3-carbonsäure-tert. butylester
N-(1-S-Carbethoxy-3-phenyl-propyl)-S-alanyl-3S-endo-2-azabicyclo[2.2.1]-heptan-3-carbonsäure-benzylester

N-(1-S-Carbethoxy-3-cyclohexylpropyl)-S-alanyl-3S-endo-2-azabicyclo[2.2.1]-heptan-3-carbonsäure-benzylester
N-(1-S-Carbethoxy-3-phenyl-propyl)-Nε-benzyloxycarbonyl-S-lysyl-3S-endo-2-azabicyclo-[2.2.1]-heptan-3-carbonsäure-tert. butylester
N-(1-S-Carbethoxy-3-phenyl-propyl)-O-ethyl-S-tyrosyl-3S-endo-2-azabicyclo[2.2.1]-heptan-3-carbonsäure-benzylester
N-(1-S-Carbethoxy-3-phenyl-propyl)-S-alanyl-2-azaspiro-[4,5]decan-3-S-carbonsäure-benzylester
N-(1-S-Carbethoxy-3-phenyl-propyl)-O-ethyl-S-tyrosyl-2-azaspiro[4,5]decan-3-S-carbonsäure-benzylester

N-(1-S-Carbethoxy-3-phenyl-propyl)-Nε-benzyloxycarbonyl-S-lysyl-2-azaspiro[4,5]decan-3-S-carbonsäure-tert. butylester
N-(1-S-Carbethoxy-3-cyclohexylpropyl)-S-alanyl-2-azaspiro[4,5]decan-3-S-carbonsäure-benzylester
N-(1-S-Carbethoxy-3-cyclohexylpropyl)-Nε-benzyloxycarbonyl-S-lysyl-2-azaspiro[4,5]-decan-3-S-carbonsäure-tert. butylester
N-(1-S-Carbethoxy-3-phenyl-propyl)-S-alanyl-2-azaspiro[4,4]nonan-3-S-carbonsäure-benzylester

N-(1-S-Carbethoxy-3-phenyl-propyl)-O-ethyl-S-tyrosyl-2-azaspiro[4,4]nonan-3-S-carbonsäure-benzylester
N-(1-S-Carbethoxy-3-phenyl-propyl)-Nε-tert.-butoxy-carbonyl-S-lysyl-2-azaspiro[4,4]nonan-3-S-carbonsäure-benzylester
N-(1-S-Carbethoxy-3-cyclohexyl-propyl)-S-alanyl-2-azaspiro[4,4]nonan-3-S-carbonsäure-benzylester

N-(1-S-Carbethoxy-3-cyclopentyl-propyl)-S-alanyl-2-azaspiro[4,4]nonan-3-S-carbonsäure-benzylester
N-(1-S-Carbethoxy-3-cyclopentyl-propyl)-Nε-tert. butoxycarbonyl-S-lysyl-2-azaspiro-[4,4]nonan-3-S-carbonsäure-benzylester
N-(1-S-Carbethoxy-3-phenyl-propyl)-S-alanyl-spiro[bicyclo[2.2.1]heptan-2,3′-pyrrolidin]-5′-S-carbonsäure-benzylester
N-(1-S-Carbethoxy-3-phenyl-propyl)-O-ethyl-S-tyrosyl-spiro[[bicyclo[2.2.1]heptan-2,3′-pyrrolidin]-5′-S-carbonsäure-benzylester
N-(1-S-Carbethoxy-3-phenyl-propyl)-N-benzyloxycarbonyl-S-lysyl-spiro[bicyclo[2.2.1]heptan-2,3′-pyrrolidin]-5′-S-carbonsäure-tert. butylester

N-(1-S-Carbethoxy-3-cyclohexyl-propyl)-S-alanyl-spiro[bicyclo[2.2.1]heptan-2,3′-pyrrolidin]-5′-S-carbonsäure-benzylester
N-(1-S-Carbethoxy-3-cyclohexyl-propyl)-Nε-tert. butoxy-carbonyl-S-lysyl-spiro[bicyclo-[2.2.1]heptan-2,3′-pyrrolidin]-5′-S-carbonsäure-benzylester
N-(1-S-Carbethoxy-3-phenyl-propyl)-S-alanyl-spiro[bicyclo[2.2.2]octan-2,3′-pyrrolidin]-5′-S-carbonsäure-benzylester
N-(1-S-Carbethoxy-3-phenyl-propyl)-O-ethyl-S-tyrosyl-spiro[bicyclo[2.2.2]octan-2,3′-pyrrolidin]-5′- S-carbonsäure-benzylester
N-(1-S-Carbethoxy-3-phenyl-propyl)-Nε-tert. butoxy-carbonyl-S-lysyl-spiro[bicyclo[2.2.2]-octan-2,3′-pyrrolidin]-5′-S-carbonsäure-benzylester

N-(1-S-Carbethoxy-3-cyclohexyl-propyl)-S-alanyl-spiro-[bicyclo[2.2.2]octan-2,3′-pyrrolidin]-5′-carbonsäure-benzylester
N-(1-S-Carbethoxy-3-phenyl-propyl)-S-alanyl-azatricyclo[4,3,0,$1^{6,9}$]decan-3-S-carbonsäure-benzylester
N-(1-S-Carbethoxy-3-phenyl-propyl)-O-ethyl-S-tyrosyl-2-azatricyclo[4,3,0,$1^{6,9}$]decan-3-S-carbonsäure-benzylester
N-(1-S-Carbethoxy-3-phenyl-propyl)-Nε-benzyloxycarbonyl-S-lysyl-2-azatricyclo[4,3,0,$1^{6,9}$]-decan-3-S-carbonsäure-butylester
N-(1-S-Carbethoxy-3-cyclohexyl-propyl)-S-alanyl-2-azatricyclo-[4,3,0,$1^{6,9}$]decan-3-S-carbonsäure-benzylester

N-(1-S-Carbethoxy-3-phenyl-propyl)-Nε-benzyloxycarbonyl-S-lysyl-2-azatricyclo[4,3,0,$1^{6,9}$]-decan-3-S-carbonsäure-tert. butylester
N-(1-S-Carbethoxy-3-phenyl-propyl)-S-alanyl-decahydrocyclohepta[b]pyrrol-2-S-carbonsäure-benzylester
N-(1-S-Carbethoxy-3-phenyl-propyl)-O-ethyl-S-tyrosyl-decahydrocyclohepta[b]pyrrol-2-S-carbonsäure-benzylester

N-(1-S-Carbethoxy-3-phenyl-propyl)-Nε-benzyloxycarbonyl-S-lysyl-decahydrocyclohepta[b]-pyrrol-2-S-carbonsäure-tert. butylester
N-(1-S-Carbethoxy-3-cyclohexyl-propyl)-S-alanyl-decahydrocyclohepta[b]pyrrol-2-S-carbonsäure-benzylester

N-(1-S-Carbethoxy-3-cyclohexyl-propyl)-Nε-tert. butoxy-carbonyl-S-lysyl-decahydrocyclohepta[b]-pyrrol-2-S-carbonsäure-benzylester
N-(1-S-Carbethoxy-3-phenyl-propyl)-S-alanyl-trans-octahydroisoindol-1-S-carbonsäure-benzylester
N-(1-S-Carbethoxy-3-phenyl-propyl)-S-alanyl-cis-octahydroisoindol-1-S-carbonsäure-benzylester
N-(1-S-Carbethoxy-3-cyclohexyl-propyl)-S-alanyl-trans-octahydroisoindol-1-S-carbonsäure-benzylester
N-(1-S-Carbethoxy-3-cyclohexyl-propyl)-S-alanyl-cis-octahydroisoindol-1-S-carbonsäure-benzylester
N-(1-S-Carbethoxy-3-phenyl-propyl)-S-alanyl-cis-octahydrocyclopenta[c]pyrrol-1-S-carbonsäure-benzylester
N-(1-S-Carbethoxy-3-cyclohexyl-propyl)-S-alanyl-cis-octahydrocyclopenta[c]pyrrol-1-S-carbonsäure-benzylester
N-(1-S-Carbethoxy-3-cyclohexyl-propyl)-Nε-tert. butoxycarbonyl-S-lysyl-cis-octahydrocyclopenta-[c]pyrrol-1-S-carbonsäure-benzylester
N-(1-S-Carbethoxy-3-phenyl-propyl)-S-alanyl-2,3,3a,4,5,7a-hexahydroindol-cis-endo-2-S-carbonsäure-tert. butylester
N-(1-S-Carbethoxy-3-cyclohexyl-propyl)-O-ethyl-S-tyrosyl-2,3,3a,4,5,7a-hexahydroindo-cis-exo-2-S-carbonsäure-tert. butylester
N-(1-S-Carbethoxy-3-cyclohexyl-propyl)-S-alanyl-2,3,3a,4,5,7a-hexahydroindol-cis-endo-2-S-carbonsäure-tert. butylester
N-(1-S-Carbethoxy-3-phenyl-propyl)-S-alanyl-thiazolidin-5-S-carbonsäure-tert. butylester
N-(1-S-Carbethoxy-3-cyclohexyl-propyl)-S-alanyl-thiazolidin-5-S-carbonsäure-tert. butylester
N-(1-S-Carbethoxy-3-phenyl-propyl)-Nε-benzyloxycarbonyllysyl-thiazolidin-5-S-carbonsäure-tert. butylester
N-(1-S-Carbethoxy-3-cyclohexyl-propyl)-Nε-benzyloxycarbonyl-2-azabicyclo[3.1.0]-hexan-3-S-carbonsäure-tert. butylester
N-(1-S-Carbethoxy-3-phenyl-propyl)-Nε-benzyloxycarbonyl-S-lysyl-2-azabicyclo[3.1.0]-hexan-cis-endo-3-S-carbonsäure-benzylester
N-(1-S-Carbethoxy-3-cyclopentylpropyl)-S-alanyl-2-azabicyclo[3.1.0]-hexan-3-carbonsäure-benzylester.

Die erfindungsgemäße Umsetzung wird vorzugsweise in neutralem oder schwach alkalischem Medium durchgeführt. Am einfachsten ist es, den pH-Wert des Mediums durch Zugabe von aliphatischen oder cycloaliphatischen tertiären Basen wie N-Methylmorpholin, N-Äthylmorpholin oder Trialkylaminen mit bis zu 6 C-Atomen pro Alkylrest einzustellen.

Als Lösungsmittel können alle in der Peptidsynthese üblichen wasserfreien, inerten Lösungsmittel, z.B. Methylenchlorid, Chloroform, Dimethylformamid, Dimethylacetamid, Dioxan, Tetrahydrofuran, 1-Methylpyrrolidon oder Dimethylsulfoxid Verwendung finden.

Die Reaktion läuft in der Regel zwischen −10 °C bis +50 °C, vorzugsweise zwischen 0 °C und Raumtemperatur hinreichend schnell ab. Leichtes Erwärmen schadet nicht. Höhere Temperaturen, etwa oberhalb 50 °C, sind wegen der Racemisierungsgefahr nicht zu empfehlen.

Der erfindungsgemäßen Alkanphosphonsäureanhydride werden vorzugsweise im Überschuß (etwa 2–2,5 Mol Alkanphosphonsäureanhydrid/Mol zu knüpfender Bindung) eingesetzt. Die Reaktion verläuft racemisierungsfrei (<2%). Bei Verwendung eines organischen oder gemischt-organischen Mediums kann die org. Phase nach der Abreaktion durch Ausschütteln mit wäßr. $KHSO_4$/$K_2SO_4$-Lösung (pH 2) und anschließend mit Soda-Bikarbonatlösung von Ausgangsstoffen sowie Verunreinigungen weitgehend befreit werden. Die entstehenden Produkte hinterbleiben als Öle nach Einengen der org. Phase und werden beispielsweise durch Hydrierung (z.B. im Falle $R^3$ = Benzyl, Nitrobenzyl) oder Säurebehandlung (z.B. wenn $SoR^3$ = $Bu^t$) in biolog. aktive Substanzen überführt.

Das erfindungsgemäße Verfahren hat erhebliche Vorteile:

Bei den dem Synthesereagentien zugrundeliegenden Säuren ist bisher keine Allergiewirkung beobachtet worden. Die Toxizität ist gering. Das Reagenz selbst, das einfach herzustellen ist, liefert insbesondere bei der Verwertung von destillierten Alkanphosphonsäureanhydriden, nach der Synthese keine schwerlöslichen Nebenprodukte, wie sie z.B. bei der häufig verwendeten Peptidknüpfung unter Verwendung von Dicyclohexylcarbodiimid auftreten.

Gegenüber den bisher beschriebenen Verfahren der Peptidsynthese unter Verwendung von Aktivierungsmitteln auf der Basis des 3- oder 5-wertigen Phosphors, wie z.B. den Peptidsynthesen nach der Phosphorazomethode (Liebigs Ann. Chem. 580, S. 68 (1953) den Synthesemethoden unter Verwendung von Diäthylchlorphosphit und Tetraäthylpyrophosphit (J. Am. chem. Soc. 74, 5304, 5307 und 5309) (1952) und der Synthesemethode unter Verwendung von Polyphosphorsäureestern (Ber. 91, (1958) S. 1073–1082 oder J. org. Chem. 26, 2534 (1961), hat das erfindungsgemäße Verfahren den Vorteil der geringeren Racemisierung bei der üblichen Verwendung von Aminosäure beziehungsweise Peptidesterhydrochloriden.

Die Verbindungen der Formel I sind Hemmstoffe des Angiotensin Converting Enzymes (ACE) beziehungsweise Zwischenprodukte bei der Herstellung von solchen Hemmstoffen und können zur Bekämpfung des Bluthochdrucks verschiedener Genese eingesetzt werden. Solche Verbindungen sind beispielsweise bekannt aus US-PS 4 344 949, US-PS 4 374 847, US-PS 4 350 704, EP-A 50 800, EP-A 31 741, EP-A 51 020, EP-A 49 658, EP-A 49 605, EP-A 29 488, EP-A 46 953 und EP-A 52 870. Sie sind weiterhin Gegenstand der deutschen Patentanmeldungen P 3 226 768.1, P 3 151 690.4, P 3 210 496.0, P 3 211 397.8, P 3 211 676.4, P 3 227 055.0, P 3 242 151.6, P 3 246 503.3 und P 3 246 757.5.

Die nachfolgenden Beispiele sollen das erfindungsgemäße Verfahren erläutern, ohne die Erfindung auf die hier stellvertretend genannten Substanzen zu beschränken.

Beispiel 1

N-(1-S-Carbethoxy-3-phenyl-propyl)-S-alanyl-cis-endo-2-azabicyclo[3.3.0]octan-3-S-carbonsäurebenzylester (N-Ethylmorpholin als Base)

31 g S,S,S-2-Azabicyclo-[3.3.0]octan-3-carbonsäurebenzylesterhydrochlorid und 30 g N-(1-S-Carbethoxy-3-phenyl-propyl)-S-alanin werden in 300 ml Methylenchlorid aufgeschlämmt. Man setzt in 1 Stunde unter Rühren 66 ml n-Propanphosphonsäureanhydrid (in Form einer 50%igen Lösung in Methylenchlorid) und 81 g N-Ethylmorpholin zu. Die Temperatur wird durch Eiskühlung zwischen 0 °C und Raumtemperatur gehalten. Nach 2 Stunden wird mit Hilfe der Dünnschichtchromatographie (Kieselgel-System: $CHCl_3$/MeOH/AcOH 50+10+3) der Umsatz kontrolliert. Man beläßt einige Stunden bei Raumtemperatur (gegebenenfalls über Nacht), engt im Vakuum ein, verdünnt den Reaktionsansatz mit Methylenchlorid (300 ml) auf ca. 0.4 l Gesamtvolumen und extrahiert 1 × mit 150 ml Wasser, 1 × mit 150 ml Wasser plus 90 ml 25%ige Kaliumhydrogensulfat, und 2 × mit je 150 ml einer wäßrigen 5% $NaHCO_3$-Lösung. Die organische Phase wird über etwas festem Natriumsulfat getrocknet und filtriert. Man engt das fast farblose Filtrat ein. Ausbeute: 95–100% d. Th. Zur Überprüfung von Ausbeute und Reinheit des Produktes kann nach beschriebenem Verfahren katalytisch entbenzyliert werden (Methanol/Pd/C). Das Reaktionsprodukt

N-(1-S-Carboethoxy-3-phenyl-propyl)-S-alanyl-cis-endo-2-azabicyclo-[3.3.0]octan-3-S-carbonsäure)

kristallisiert aus Äther in 80–90%iger Ausbeute (bezogen auf S,S,S-2-Azabicyclo[3.3.0]octan-3-carbonsäure-benzylester-hydrochlorid).

Schmp.: 109 °C; $[\alpha]_D^{24} = +15{,}7°$ (C = 1, Methanol).

Beispiel 2

N-(1-S-Carboethoxy-3-phenyl-propyl)-S-alanyl-cis-endo-2-azabicyclo[3.3.0]octan-3-S-carbonsäurebenzylester (mit Triethylamin als Base)

20 g S,S,S-2-Azabicyclo[3.3.0]octan-3-carbonsäure-benzylesterhydrochlorid werden in 200 ml Methylenchlorid aufgeschlämmt. Dazu werden 66 ml Triethylamin und 20 g N-(1-S-Carboethoxy-3-phenyl-propyl)-S-alanin zugegeben. Dann tropft man unter Eiskühlung und Rühren 44 ml n-Propanphosphonsäureanhydrid (in Form einer 50%igen Lösung in Methylenchlorid) zu. Die Temperatur der Reaktionslösung liegt zwischen 0 °C und 25 °C. Es wird 3 Stunden nachgerührt, danach mit 200 ml Methylenchlorid verdünnt, mit 200 ml Wasser ausgerührt, vom Wasser abgetrennt und nacheinander mit 200 ml Wasser plus 100 ml 25%ige Kaliumhydrogensulfat, dann mit 150 ml Wasser und schließlich mit 100 ml Natriumhydrogencarbonat gewaschen. Es wird die Methylenchloridlösung getrocknet über festem Magnesiumsulfat und filtriert. Man engt das farblose Filtrat ein. Ausbeute: ca. 95% des Benzylesters.

Zur Überprüfung von Ausbeute und Reinheit des Produktes kann nach beschriebenem Verfahren katalytisch entbenzyliert werden (Methanol/Pd/C). Das Reaktionsprodukt

N-(1-S-Carboethoxy-3-phenylpropyl)-S-alanyl-cis-endo-2-azabicyclo[3.3.0]octan-3-S-carbonsäure

kristallisiert aus Diisopropylether in 90% Ausbeute bezogen auf S,S,S-2-Azabicyclo[3.3.0]octan-3-carbonsäure-benzylhydrochlorid.

## Patentansprüche

1. Verfahren zur Herstellung von Verbindungen der Formel I

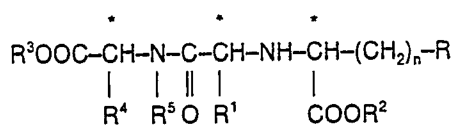

(I)

in welcher

n = 1 oder 2 ist,

R = Wasserstoff,

einen gegebenenfalls substituierten aliphatischen Rest mit 1 bis 8 C-Atomen,

einen gegebenenfalls substituierten alicyclischen Rest mit 3–9 C-Atomen,

einen gegebenenfalls substituierten aromatischen Rest mit 6–12 C-Atomen,

einen gegebenenfalls substituierten araliphatischen Rest mit 7–14 C-Atomen,

einen gegebenenfalls substituierten alicyclischaliphatischen Rest mit 7–14 C-Atomen,

einen Rest $OR^a$ oder $SR^a$, worin

$R^a$ für einen gegebenenfalls substituierten aliphatischen Rest mit 1–4 C-Atomen, für einen gegebenenfalls substituierten aromatischen Rest mit 6–12 C-Atomen oder einen gegebenenfalls substituierten heteroaromatischen Rest mit 6–12 Ringatomen steht,

$R^1$ Wasserstoff, einen gegebenenfalls substituierten aliphatischen Rest mit 1 bis 6 C-Atomen,

einen gegebenenfalls substituierten alicyclischen Rest mit 3–9 C-Atomen,

einen gegebenenfalls substituierten alicyclischaliphatischen Rest mit 4–13 C-Atomen,

einen gegebenenfalls substituierten aromatischen Rest mit 6–12 C-Atomen,

einen gegebenenfalls substituierten araliphatischen Rest mit 7–16 C-Atomen,

einen gegebenenfalls substituierten heteroaromatischen Rest mit 5–12 Ringatomen oder

die erforderlichenfalls geschützte Seitenkette einer natürlich vorkommenden α-Aminosäure bedeuten,

$R^2$ und $R^3$ gleich oder verschieden sind und Wasserstoff, einen gegebenenfalls substituierten aliphatischen Rest mit 1–6 C-Atomen,

einen gegebenenfalls substituierten alicyclischen Rest mit 3–9 C-Atomen,
einen gegebenenfalls substituierten aromatischen Rest mit 6–12 C-Atomen,
einen gegebenenfalls substituierten araliphatischen Rest mit 7–16 C-Atomen bedeuten und
$R^4$ und $R^5$ zusammen mit den sie tragenden Atomen ein heterocyclisches, mono-, bi-, oder tricyclisches Ringsystem mit 5 bis 15 C-Atomen bilden, dadurch gekennzeichnet, daß man Verbindungen der Formel II

$$\begin{array}{l} HO\!-\!C\!-\!CH\!-\!NH\!-\!CH\!-\!(CH_2)_n\!-\!R \\ \;\;\;\; \| \;\;\; | \qquad\quad\;\; | \\ \;\;\;\; O \;\; R^1 \qquad\; COOR^2 \end{array} \qquad (II),$$

in welcher n, R, $R^1$ und $R^2$ die oben definierten Bedeutungen haben, in Gegenwart von Alkanphosphonsäureanhydriden mit Verbindungen der Formel III

$$\begin{array}{l} R^3OOC\!-\!CH\!-\!NH \\ \qquad\quad\;\; | \quad\; | \\ \qquad\quad\; R^4 \;\; R^5 \end{array} \qquad (III)$$

in welcher $R^3$, $R^4$ und $R^5$ die oben definierten Bedeutungen haben, umsetzt, gegebenenfalls zum Schutz anderer funktioneller Gruppen eingeführte Reste abspaltet und gegebenenfalls freie Carboxygruppen in an sich bekannter Weise verestert.

2. Verfahren zur Herstellung von Verbindungen der Formel I

$$\begin{array}{l} \qquad\quad\;\; * \qquad\; * \qquad * \\ R^3OOC\!-\!CH\!-\!N\!-\!C\!-\!CH\!-\!NH\!-\!CH\!-\!(CH_2)_n\!-\!R \\ \qquad\quad\;\; | \quad\; | \;\; \| \;\; | \qquad\quad\; | \\ \qquad\quad\; R^4 \;\; R^5 \, O \; R^1 \qquad COOR^2 \end{array} \qquad (I)$$

worin n, R, $R^a$, $R^1$, $R^2$ und $R^3$ die im Anspruch 1 angegebene Bedeutung haben und $R^4$ und $R^5$ zusammen mit den sie tragenden Atomen für ein gegebenenfalls substituiertes System aus der Reihe Pyrrolidon, Piperidin, Tetrahydroisochinolin, Decahydroisochinolin, Octahydroindol, Octahydrocyclopenta[b]pyrrol, 2-Aza-bicyclo[2.2.2]octan, 2-Azabicyclo[2.2.1]heptan, 2-Azaspiro[4.5]decan, 2-Azaspiro[4.4]nonan, Spiro[(bicyclo[2.2.1]heptan)-2,3-pyrrolidin], Spiro[(bicyclo[2.2.2]octan)-2,3-pyrrolidin], 2-Azatricyclo[$4,3,0,1^{6,9}$]decan, Decahydrocyclohepta[b]pyrrol, Octahydroisoindol, Octahydrocyclopenta[c]pyrrol, 2,3,3a,4,5,7a-Hexahydroindol, Tetrahydrothiazol und 2-Azabicyclo[3.1.0]hexan stehen, dadurch gekennzeichnet, daß man Verbindungen der Formel II

$$\begin{array}{l} HO\!-\!C\!-\!CH\!-\!NH\!-\!CH\!-\!(CH_2)_n\!-\!R \\ \;\;\;\; \| \;\;\; | \qquad\quad\;\; | \\ \;\;\;\; O \;\; R^1 \qquad\; COOR^2 \end{array} \qquad (II),$$

in welcher n, R, $R^1$ und $R^2$ die oben definierten Bedeutungen haben, in Gegenwart von Alkanphosphonsäureanhydriden
mit Verbindungen der Formel III

$$\begin{array}{l} R^3OOC\!-\!CH\!-\!NH \\ \qquad\quad\;\; | \quad\; | \\ \qquad\quad\; R^4 \;\; R^5 \end{array} \qquad (III)$$

in welcher $R^3$, $R^4$ und $R^5$ die oben definierten Bedeutungen haben, umsetzt, gegebenenfalls zum Schutz anderer funktioneller Gruppen eingeführte Reste abspaltet und gegebenenfalls freie Carboxygruppen in an sich bekannter Weise verestert.

3. Verfahren gemäß einem der Ansprüche 1 oder 2, dadurch gekennzeichnet, daß Verbindungen der Formel I hergestellt werden, in welcher
n = 1 oder 2 ist
R = Wasserstoff,
Alkyl mit 1–8 C-Atomen,
Alkenyl mit 2–6 C-Atomen,
Cycloalkyl mit 3–9 C-Atomen,
Aryl mit 6–12 C-Atomen,
das durch ($C_1$–$C_4$)-Alkyl, ($C_1$–$C_4$)-Alkoxy, Hydroxy, Halogen, Nitro, Amino, Aminomethyl, ($C_1$–$C_4$)-Alkylamino, Di-($C_1$–$C_4$)-Alkylamino, ($C_1$–$C_4$)-Acylamino, Methylendioxy, Carboxy, Cyano und/oder Sulfamoyl mono-, di- oder trisubstituiert sein kann,
Alkoxy mit 1–4 C-Atomen,
Aryloxy mit 6–12 C-Atomen,
das wie oben bei Aryl beschrieben substituiert sein kann,
mono bzw. bicyclisches Heteroaryloxy mit 5–7 bzw. 8–10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,
Amino-($C_1$–$C_4$)-alkyl,
($C_1$–$C_4$)-Alkynoylamino-($C_1$–$C_4$)-alkyl,
($C_7$–$C_{13}$)-Aroylamino-($C_1$–$C_4$)-alkyl,
($C_1$–$C_4$)-Alkoxy-carbonylamino-($C_1$–$C_4$)-alkyl,
($C_6$–$C_{12}$)-Aryl-($C_1$–$C_4$)-alkoxycarbonylamino-($C_1$–$C_4$)-alkyl,
($C_6$–$C_{12}$)-Aryl-($C_1$–$C_4$)-alkylamino-($C_1$–$C_4$)-alkyl,
($C_1$–$C_4$)-Alkylamino-($C_1$–$C_4$)-alkyl,
Di-($C_1$–$C_4$)-alkylamino-($C_1$–$C_4$)-alkyl,
Guanidino-($C_1$–$C_4$)-alkyl,
Imidazolyl, Indolyl,
($C_1$–$C_4$)-Alkylthio,
($C_1$–$C_4$)-Alkylthio-($C_1$–$C_4$)-alkyl,
($C_6$–$C_{12}$)-Arylthio-($C_1$–$C_4$)-alkyl,
das im Arylteil wie oben bei Aryl beschrieben, substituiert sein kann,
($C_6$–$C_{12}$)-Aryl-($C_1$–$C_4$)-alkylthio,
das im Arylteil wie oben bei Aryl beschrieben, substituiert sein kann,
Carboxy-($C_1$–$C_4$)-alkyl,
Carboxy, Carbamoyl,
Carbamoyl-($C_1$–$C_4$)-alkyl,
($C_1$–$C_4$)-Alkoxy-carbonyl-($C_1$–$C_4$)-alkyl,
($C_6$–$C_{12}$)-Aryloxy-($C_1$–$C_4$)-alkyl,

das im Arylteil wie oben bei Aryl beschrieben, substituiert sein kann oder
$(C_6–C_{12})$-Aryl-$(C_1–C_4)$-alkoxy-
das im Arylteil wie oben bei Aryl beschrieben, substituiert sein kann,
$R^1$ Wasserstoff,
Alkyl mit 1–6 C-Atomen,
Alkenyl mit 2–6 C-Atomen,
Alkinyl mit 2–6 C-Atomen,
Cycloalkyl mit 3–9 C-Atomen,
Cycloalkenyl mit 5–9 C-Atomen,
$(C_3–C_9)$-Cycloalkyl-$(C_1–C_4)$-alkyl,
$(C_5–C_9)$-Cycloalkenyl-$(C_1–C_4)$-alkyl,
gegebenenfalls teilhydriertes Aryl mit 6–12 C-Atomen, das wie oben bei R beschrieben, substituiert sein kann,
$(C_6–C_{12})$-Aryl-$(C_1–C_4)$-alkyl oder $(C_7–C_{13})$-Aroyl-$(C_1–C_2)$-alkyl,
die beide wie das vorstehende Aryl substituiert sein können
mono- bzw. bicyclisches, gegebenenfalls teilhydriertes Heteroaryl mit 5–7 bzw. 8–10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette natürlich vorkommenden α-Aminosäure $R^1$–$CH(NH_2)$–COOH bedeuten,
$R^2$ und $R^3$ gleich oder verschieden sind und
Wasserstoff,
Alkyl mit 1–6 C-Atomen,
Alkenyl mit 2–6 C-Atomen,
Di-$(C_1–C_4)$-alkylamino-$(C_1–C_4)$-alkyl,
$(C_1–C_5)$-Alkanoyloxy-$(C_1–C_4)$-alkyl,
$(C_1–C_6)$-Alkoxy-carbonyloxy-$(C_1–C_4)$-alkyl,
$(C_7–C_{13})$-Aroyloxy-$(C_1–C_4)$-alkyl,
$(C_6–C_{12})$-Aryloxycarbonyloxy-$(C_1–C_4)$-alkyl,
Aryl mit 6–12 C-Atomen,
$(C_6–C_{12})$-Aryl-$(C_1–C_4)$-alkyl,
$(C_3–C_9)$-Cycloalkyl oder
$(C_3–C_9)$-Cycloalkyl-$(C_1–C_4)$-alkyl
bedeuten und
$R^4$ und $R^5$ die oben angegebene Bedeutung haben, wobei während der Umsetzung gegebenenfalls in den Resten R bis $R^5$ vorhandene freie Amino-, Alkylamino-, Hydroxy-, Carboxy-, Mercapto- und/oder Guanidinofunktionen in an sich bekannter Weise blockiert werden.

4. Verfahren gemäß einem der Ansprüche 1 bis 3, dadurch gekennzeichnet, daß in Gegenwart von n-Propanphosphonsäureanhydrid gearbeitet wird.

5. Verfahren gemäß einem der Ansprüche 1 bis 4, dadurch gekennzeichnet, daß die Umsetzung in einem inerten organischen Lösungsmittel in Gegenwart eines tert. organischen Amines durchgeführt wird.

6. Verfahren gemäß einem der Ansprüche 1 bis 5, dadurch gekennzeichnet, daß
[S,S,S,S,S]-N-[(1-Carbethoxy-3-phenyl-propyl)-alanyl]-octahydroindol-2-carbonsäure-benzylester
hergestellt wird.

7. Verfahren gemäß einem der Ansprüche 1 bis 5, dadurch gekennzeichnet, daß
N-[(1(S)-Carbethoxy-3-phenyl-propyl)-(S)-alanyl]-3aR, 7aS-octahydroindol-2-(S)-carbonsäure-benzylester
hergestellt wird.

8. Verfahren gemäß einem der Ansprüche 1 bis 5, dadurch gekennzeichnet, daß
[S,S,S,S,S]-N-[(1-Carbethoxy-3-phenyl-propyl)-alanyl]-decahydroisochinolin-3-carbonsäure-benzylester
hergestellt wird.

9. Verfahren gemäß einem der Ansprüche 1 bis 5, dadurch gekennzeichnet, daß
[S,S,S]-N-[(1-Carbethoxy-3-phenyl-propyl)-alanyl]-tetrahydroisochinolin-3-carbonsäure-benzylester
hergestellt wird.

10. Verfahren gemäß einem der Ansprüche 1 bis 5, dadurch gekennzeichnet, daß
(S,S,S,S,S)-N-(1-Carbethoxy-3-phenyl-propyl)-alanyl-2-azabicyclo[3.3.0]octan-3-carbonsäure-benzylester
hergestellt wird.

11. Verfahren gemäß einem der Ansprüche 1 bis 5, dadurch gekennzeichnet, daß
N-(1-S-Carbethoxy-3-phenyl-propyl)-S-alanyl-cis-endo-2-azabicyclo[3.1.0]hexan-3-S-carbonsäurebenzylester
hergestellt wird.

12. Verfahren gemäß einem der Ansprüche 1 bis 5, dadurch gekennzeichnet, daß
N-(1-S-Carbethoxy-3-phenyl-propyl)-S-alanyl-cis-endo-2,3,3a,4,5,7a-hexahydroindol-2-S-carbonsäure-benzylester
hergestellt wird.

13. Verfahren gemäß einem der Ansprüche 6 bis 12, dadurch gekennzeichnet, daß anstelle der Benzylester die entsprechenden tert. Butylester oder daß andere hydrogenolytisch, sauer oder basisch spaltbare Ester hergestellt werden.

**Claims**

1. A process for the preparation of a compound of the formula I

$$R^3OOC–\underset{R^4}{\overset{*}{C}H}–\underset{R^5}{N}–\underset{O}{\overset{\|}{C}}–\underset{R^1}{\overset{*}{C}H}–NH–\underset{COOR^2}{\overset{*}{C}H}–(CH_2)_n–R \quad (I)$$

in which
n is 1 or 2,
R denotes hydrogen,
an optionally substituted aliphatic radical having 1 to 8 carbon atoms
an optionally substituted alicyclic radical having 3–9 carbon atoms,
an optionally substituted aromatc radical having 6–12 carbon atoms,
an optionally substituted araliphatic radical having 7–14 carbon atoms,

an optionally substituted alicyclic-aliphatic radical having 7–14 carbon atoms,
or a radical $OR^a$ or $SR^a$, in which

$R^a$ represents an optionally substituted aliphatic radical having 1–4 carbon atoms, an optionally substituted aromatic radical having 6–12 carbon atoms or an optionally substituted heteroaromatic radical having 5–12 ring atoms,

$R^1$ denotes hydrogen, an optionally substituted aliphatic radical having 1 to 6 carbon atoms,
an optionally substituted alicyclic radical having 3–9 carbon atoms,
an optionally substituted alicyclic-aliphatic radical having 4–13 carbon atoms,
an optionally substituted aromatic radical having 6–12 carbon atoms,
an optionally substituted araliphatic radical having 7–16 carbon atoms,
an optionally substituted heteroaromatic radical having 5–12 ring atoms,
or the side chain, protected when necessary, of a naturally occurring α-amino acid,

$R^2$ and $R^3$ are identical or different and denote hydrogen, an optionally substituted aliphatic radical having 1–6 carbon atoms,
an optionally substituted alicyclic radical having 3–9 carbon atoms,
an optionally substituted aromatic radical having 6–12 carbon atoms,
or an optionally substituted araliphatic radical having 7–16 carbon atoms, and

$R^4$ and $R^5$, together with the atoms bearing them, form a heterocyclic, monocyclic, bicyclic or tricyclic ring system having 5 to 15 carbon atoms, which process comprises reacting a compound of the formula II

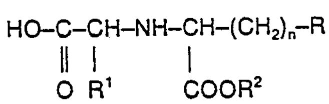 (II),

in which

n, R, $R^1$ and $R^2$ have the meanings defined above, in the presence of alkanephosphonic anhydrides, with a compound of the formula III

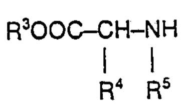 (III)

in which

$R^3$, $R^4$ and $R^5$ have the meanings defined above, where appropriate eliminating radicals which have been introduced to protect other functional groups and, where appropriate, esterifying free carboxyl groups in a manner known per se.

2. A process for the preparation of a compound of the formula I

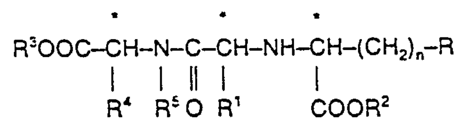 (I)

in which

n, R, $R^a$, $R^1$, $R^2$ and $R^3$ have the meaning given in claim 1, and $R^4$ and $R^5$, together with the atoms bearing them, represent an optionally substituted system from the series comprising pyrrolidine, piperidine, tetrahydroisoquinoline, decahydroisoquinoline, octahydroindole, octahydrocyclopenta[b]pyrrole, 2-azabicyclo[2.2.2]octane, 2-azabicyclo[2.2.1]heptane, 2-azaspiro[4.5]decan, 2-azaspiro[4.4]nonane, spiro[(bicyclo[2.2.1]heptane)-2,3-pyrrolidine], spiro[(bicyclo[2.2.2]octane)-2,3-pyrrolidine], 2-azatricyclo[$4.3.0.1^{6,9}$]-decane, decahydrocyclohepta[b]pyrrole, octahydroisoindole, octahydrocyclopenta[c]pyrrole, 2,3,3a,4,5,7a-hexahydroindole, tetrahydrothiazole and 2-azabicyclo[3.1.0]hexane, which comprises reacting a compound of the formula II

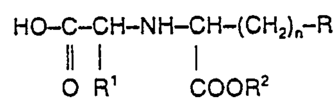 (II),

in which

n, R, $R^1$ and $R^2$ have the meanings defined above, in the presence of alkanephosphonic anhydrides, with a compound of the formula II

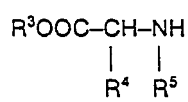 (III)

in which

$R^3$, $R^4$ and $R^5$ have the meanings defined above, where appropriate eliminating radicals which have been introduced to protect other functional groups and, where appropriate, esterifying free carboxyl groups in a manner known per se.

3. The process as claimed in one of claims 1 or 2, in which is prepared a compound of the formula I in which

n is 1 or 2,

R denotes hydrogen,
alkyl having 1–8 carbon atoms,
alkenyl having 2–6 carbon atoms
cycloalkyl having 3–9 carbon atoms,
aryl having 6–12 carbon atoms,
which can be monosubstituted, disubstituted or trisubstituted by ($C_1$–$C_4$)-alkyl, ($C_1$–$C_4$)-alkoxy, hydroxyl, halogen, nitro, amino, aminomethyl, ($C_1$–$C_4$)-alkylamino, di-($C_1$–$C_4$)-alkylamino, ($C_1$–$C_4$)-acylamino, methylenedioxy, carboxyl, cyano, and/or sulfamoyl,
alkoxy having 1–4 carbon atoms,
aryloxy having 6–12 carbon atoms,
which can be substituted as described above for aryl,

monocyclic or bicyclic heteroaryloxy having 5–7 or 8–10 ring atoms respectively, 1 or 2 of these ring atoms being sulfur or oxygen atoms and/or 1 to 4 ring atoms being nitrogen, which can be substituted as described above for aryl,
amino-($C_1$–$C_4$)-alkyl,
($C_1$–$C_4$)-alkanoylamino-($C_1$–$C_4$)-alkyl,
($C_7$–$C_{13}$)-aroylamino-($C_1$–$C_4$)-alkyl,
($C_1$–$C_4$)-alkoxycarbonylamino-($C_1$–$C_4$)-alkyl,
($C_6$–$C_{12}$)-aryl-($C_1$–$C_4$)-alkoxycarbonylamino-($C_1$–$C_4$)-alkyl,
($C_6$–$C_{12}$)-aryl-($C_1$–$C_4$)-alkylamino-($C_1$–$C_4$)-alkyl,
($C_1$–$C_4$)-alkylamino-($C_1$–$C_4$)-alkyl,
di-($C_1$–$C_4$)-alkylamino-($C_1$–$C_4$)-alkyl,
guanidino-($C_1$–$C_4$)-alkyl,
imidazolyl, indolyl,
($C_1$–$C_4$)-alkylthio,
($C_1$–$C_4$)-alkylthio-($C_1$–$C_4$)-alkyl,
($C_6$–$C_{12}$)-arylthio-($C_1$–$C_4$)-alkyl,
which can be substituted in the aryl moiety as described above for aryl,
($C_6$–$C_{12}$)-aryl-($C_1$–$C_4$)-alkylthio,
which can be substituted in the aryl moiety as described above for aryl,
carboxy-($C_1$–$C_4$)-alkyl,
carboxyl, carbamoyl,
carbamoyl-($C_1$–$C_4$)-alkyl,
($C_1$–$C_4$)-alkoxycarbonyl-($C_1$–$C_4$)-alkyl,
($C_6$–$C_{12}$)-aryloxy-($C_1$–$C_4$)-alkyl,
which can be substituted in the aryl moiety as described above for aryl, or
($C_6$–$C_{12}$)-aryl-($C_1$–$C_4$)-alkoxy,
which can be substituted in the aryl moiety as described above for aryl, or
$R^1$ denotes hydrogen,
alkyl having 1–6 carbon atoms,
alkenyl having 2–6 carbon atoms,
alkynyl having 2–6 carbon atoms,
cycloalkyl having 3–9 carbon atoms,
cycloalkenyl having 5–9 carbon atoms,
($C_3$–$C_9$)-cycloalkyl-($C_1$–$C_4$)-alkyl,
($C_5$–$C_9$)-cycloalkenyl-($C_1$–$C_4$)-alkyl,
optionally partially hydrogenated aryl having 6–12 carbon atoms,
which can be substituted as described above for R, ($C_6$–$C_{12}$)-aryl-($C_1$–$C_4$)-alkyl or ($C_7$–$C_{13}$)-aroyl-($C_1$ or $C_2$)-alkyl, both of which can be substituted as the previous aryl, monocyclic or bicyclic, optionally partially hydrogenated, heteroaryl having 5–7 or 8–10 ring atoms respectively, 1 or 2 of these ring atoms being sulfur or oxygen atoms and/or 1 to 4 ring atoms being nitrogen atoms, which can be substituted as the previous aryl, or the optionally protected side chain of a naturally occurring α-amino acid $R^1$–CH($NH_2$)–COOH,
$R^2$ and $R^3$ are identical or different and denote hydrogen,
alkyl having 1–6 carbon atoms,
alkenyl having 2–6 carbon atoms,
di-($C_1$–$C_4$)-alkylamino-($C_1$–$C_4$)-alkyl,
($C_1$–$C_5$)-alkanoyloxy-($C_1$–$C_4$)-alkyl,
($C_1$–$C_6$)-alkoxycarbonyloxy-($C_1$–$C_4$)-alkyl,
($C_7$–$C_{13}$)-aroyloxy-($C_1$–$C_4$)-alkyl,
($C_6$–$C_{12}$)-aryloxycarbon-yloxy-($C_1$–$C_4$)-alkyl, aryl having 6–12 carbon atoms,
($C_6$–$C_{12}$)-aryl-($C_1$–$C_4$)-alkyl,
($C_3$–$C_9$)-cycloalkyl or
($C_3$–$C_9$)-cycloalkyl-($C_1$–$C_4$)-alkyl, and
$R^4$ and $R^5$ have the abovementioned meaning, during the reaction the free amino, alkylamino, hydroxyl, carboxyl, mercapto and/or guanidino groups present, where appropriate, in the radicals R to $R^5$ being protected in a manner known per se.

4. The process as claimed in one of claims 1 to 3, which is carried out in the presence of n-propanephosphonic anhydride.

5. The process as claimed in one of claims 1 to 4, wherein the reaction is carried out in an inert organic solvent in the presence of a tertiary organic amine.

6. The process as claimed in one of claims 1 to 5, wherein benzyl
[S,S,S,S,S]-N-[(1-carboethoxy-3-phenyl-propyl)alanyl]octahydroindole-2-carboxylate
is prepared.

7. The process as claimed in one of claims 1 to 5, wherein benzyl
N-[(1(S)-carboethoxy-3-phenyl-propyl)-(S)-alanyl]-3aR,7aS-octahydroindole-2-(S)-carboxylate
is prepared.

8. The process as claimed in one of claims 1 to 5, wherein benzyl
[S,S,S,S,S]-N-[(1-carboethoxy-3-phenyl-propyl)alanyl]decahydroisoquinoline-3-carboxylate
is prepared.

9. The process as claimed in one of claims 1 to 5, wherein benzyl
[S,S,S]-N-[(1-carboethoxy-3-phenyl-propyl)-alanyl]tetrahydroisoquinoline-3-carboxylate
is prepared.

10. The process as claimed in one of claims 1 to 5, wherein benzyl
(S,S,S,S,S)-N-(1-carboethoxy-3-phenyl-propyl)-alanyl-2-azabicyclo[3.3.0]-octane-3-carboxylate
is prepared.

11. The process as claimed in one of claims 1 to 5, wherein benzyl
N-(1-S-carboethoxy-3-phenyl-propyl)-S-alanyl-cis-endo-2-azabicyclo[3.1.0]-hexane-3-S-carboxylate
is prepared.

12. The process as claimed in one of claims 1 to 5, wherein benzyl
N-(1-S-carboethoxy-3-phenyl-propyl)-S-alanyl-cis-endo-2,3,3a,4,5,7a-hexahydroindole-2-S-carboxylate
is prepared.

13. The process as claimed in one of claims 6 to 12, wherein, in place of the benzyl ester, the corresponding tert.-butyl ester or other esters which can be cleaved by hydrogenolysis, acid or base are prepared.

**Revendications**

1. Procédé pour la préparation de composés de formule I

```
     •     •     •
R³OOC–CH–N–C–CH–NH–CH–(CH₂)ₙ–R        (I)
      |  | ‖  |     |
      R⁴ R⁵O  R¹    COOR²
```

dans laquelle

n = 1 ou 2,

R représente un atome d'hydrogène, un radical aliphatique éventuellement substitué ayant de 1 à 8 atomes de carbone, un radical alicyclique éventuellement substitué ayant de 3 à 9 atomes de carbone, un radical aromatique éventuellement substitué ayant de 6 à 12 atomes de carbone, un radical araliphatique éventuellement substitué ayant de 7 à 14 atomes de carbone, un radical alicyclique-aliphatique éventuellement substitué ayant de 7 à 14 atomes de carbone, un radical $OR^a$ ou $SR^a$, dans lequel

$R^a$ représente un reste aliphatique éventuellement substitué ayant de 1 à 4 atomes de carbone, un reste aromatique éventuellement substitué ayant de 6 à 12 atomes de carbone ou un reste hétéroaromatique éventuellement substitué ayant de 5 à 12 atomes nucléaires,

$R^1$ représente un atome d'hydrogène, un radical aliphatique éventuellement substitué ayant de 1 à 6 atomes de carbone, un radical alicyclique éventuellement substitué ayant de 3 à 9 atomes de carbone, un radical alicyclique-aliphatique éventuellement substitué ayant de 4 à 13 atomes de carbone, un radical aromatique éventuellement substitué ayant de 6 à 12 atomes de carbone, un radical araliphatique éventuellement substitué ayant de 7 à 16 atomes de carbone, un radical hétéroaromatique éventuellement substitué ayant de 5 à 12 atomes nucléaires, ou la chaîne latérale, protégé si nécessaire, d'un α-aminoacide existant dans la nature,

$R^2$ et $R^3$ sont identiques ou différents, et représentent un atome d'hydrogène, un radical aliphatique éventuellement substitué ayant de 1 à 6 atomes de carbone, un radical alicyclique éventuellement substitué ayant de 3 à 9 atomes de carbone, un radical aromatique éventuellement substitué ayant de 6 à 12 atomes de carbone, un radical araliphatique éventuellement substitué ayant de 7 à 16 atomes de carbone, et

$R^4$ et $R^5$ forment, conjointement avec les atomes qui les portent, un système cyclique mono-, bi- ou tricyclique hétérocyclique ayant de 5 à 15 atomes de carbone, caractérisé en ce que l'on fait réagir des composés de formule II

```
HO–C–CH–NH–CH–(CH₂)ₙ–R        (II),
   ‖  |     |
   O  R¹    COOR²
```

dans laquelle

n, R, $R^1$ et $R^2$ ont les significations données plus haut, en présence d'anhydrides alcanephosphoniques, avec des composés de formule III

```
R³OOC–CH–NH        (III)
      |  |
      R⁴ R⁵
```

dans laquelle

$R^3$, $R^4$ et $R^5$ ont les significations données plus haut, éventuellement on élimine des radicaux introduits pour la protection d'autres groupes fonctionnels et éventuellement on estérifie d'une façon connue en soi des groupes carboxy libres.

2. Procédé pour la préparation de composés de formule I

```
     •     •     •
R³OOC–CH–N–C–CH–NH–CH–(CH₂)ₙ–R        (I)
      |  | ‖  |     |
      R⁴ R⁵O  R¹    COOR²
```

dans laquelle

n, R, $R^a$, $R^1$, $R^2$ et $R^3$ ont les significations données dans la revendication 1, et

$R^4$ et $R^5$ forment, conjointement avec les atomes qui les portent, un système éventuellement substitué choisi parmi les systèmes pyrrolidine, pipéridine, tétrahydroisoquinoléine, décahydroisoquinoléine, octahydroindole, octahydrocyclopenta[b]pyrrole, 2-azabicyclo[2.2.2]octane, 2-azabicyclo[2.2.1]heptane, 2-azaspiro[4.5]décane, 2-azaspiro[4.4]nonane, spiro[(bicyclo[2.2.1]heptane)-2,3-pyrrolidine], spiro[(bicyclo[2.2.2]octane)-2,3-pyrrolidine], 2-azatricyclo[$4.3.0.1^{6,9}$]-décane, décahydrocyclohepta[b]pyrrole, octahydroisoindole, octahydrocyclopenta[c]pyrrole, 2,3,3a,4,5,7ahexahydroindole, tétrahydrothiazole et 2-azabicyclo[3.1.0]hexane, caractérisé en ce que l'on fait réagir des composés de formule II

```
HO–C–CH–NH–CH–(CH₂)ₙ–R        (II),
   ‖  |     |
   O  R¹    COOR²
```

dans laquelle

n, R, $R^1$ et $R^2$ ont les significations données plus haut, en présence d'anhydrides alcanephosphoniques, avec des composés de formule III

```
R³OOC–CH–NH        (III)
      |  |
      R⁴ R⁵
```

dans laquelle

$R^3$, $R^4$ et $R^5$ ont les significations données plus haut, éventuellement on élimine des radicaux introduits pour la protection d'autres groupes fonctionnels et éventuellement on estérifie d'une façon connue en soi des groupes carboxy libres.

3. Procédé selon l'une des revendications 1 ou 2, caractérisé en ce que l'on prépare des composés de formule I dans lesquels

n = 1 ou 2,

R représente un atome d'hydrogène,
un radical alkyle ayant de 1 à 8 atomes de carbone,
un radical alcényle ayant de 2 à 6 atomes de carbone,
un radical cycloalkyle ayant de 3 à 9 atomes de carbone,

un radical aryle ayant de 6 à 12 atomes de carbone, qui peut être mono-, di- ou trisubstitué par un ou des atomes d'halogène ou groupe(s) alkyle en $C_1$–$C_4$, alcoxy en $C_1$–$C_4$, hydroxy, nitro, amino, aminométhyle, alkyl($C_1$–$C_4$)-amino, dialkyl($C_1$–$C_4$)-amino, acyl($C_1$–$C_4$)-amino, méthylènedioxy, carboxy, cyano et/ou sulfamoyle,
un radical alcoxy ayant de 1 à 4 atomes de carbone,

un radical aryloxy ayant de 6 à 12 atomes de carbone, qui peut être substitué comme décrit plus haut pour le radical aryle,
un radical hétéroaryloxy mono- ou bycyclique ayant de 5 à 7 ou de 8 à 10 atomes nucléaires, dont 1 ou 2 atomes nucléaires sont un ou des atomes de soufre ou d'oxygène et/ou de 1 à 4 atomes nucléaires représentent un ou des atomes d'azote,

qui peut être substitué comme décrit plus haut pour le radical aryle,
un radical aminoalkyle en $C_1$–$C_4$,
alcanoyl($C_1$–$C_4$)-amino-alkyle($C_1$–$C_4$),
aroyl($C_7$–$C_{13}$)-amino-alkyle($C_1$–$C_4$),
alcoxy($C_1$–$C_4$)-carbonylamino-alkyle($C_1$–$C_4$),
aryl($C_6$–$C_{12}$)-alcoxy($C_1$–$C_4$)-carbonylamino-alkyle($C_1$–$C_4$),
aryl($C_6$–$C_{12}$)-alkyl($C_1$–$C_4$)-amino-alkyle($C_1$–$C_4$),
alkyl($C_1$–$C_4$)-amino-alkyle($C_1$–$C_4$),
dialkyl($C_1$–$C_4$)-amino-alkyle($C_1$–$C_4$),
guanidino-alkyle($C_1$–$C_4$),
imidazolyle, indolyle,
alkyl($C_1$–$C_4$)-thio,
alkyl($C_1$–$C_4$)-thio-alkyle($C_1$–$C_4$),
aryl($C_6$–$C_{12}$)-thio-alkyle($C_1$–$C_4$)
qui peut être substitué sur le fragment aryle comme décrit plus haut pour le radical aryle,
aryl($C_6$–$C_{12}$)-alkyl($C_1$–$C_4$)-thio qui peut être substitué sur le fragment aryle comme décrit plus haut pour le radical aryle,
carboxy-alkyle($C_1$–$C_4$),
carboxy, carbamoyle,
carbamoyl-alkyle($C_1$–$C_4$),
alcoxy($C_1$–$C_4$)-carbonyl-alkyle($C_1$–$C_4$),
aryloxy($C_6$–$C_{12}$)-alkyle($C_1$–$C_4$) qui peut être substitué sur le fragment aryle comme décrit plus haut pour le radical aryle, ou
aryl($C_6$–$C_{12}$)-alcoxy($C_1$–$C_4$) qui peut être substitué sur le fragment aryle comme décrit plus haut pour le radical aryle,

$R^1$ représente un atome d'hydrogène,
un radical alkyle ayant de 1 à 6 atomes de carbone,
alcényle ayant de 2 à 6 atomes de carbone,
alcynyle ayant de 2 à 6 atomes de carbone,
cycloalkyle ayant de 3 à 9 atomes de carbone,
cycloalcényle ayant de 5 à 9 atomes de carbone,
cycloalkyl($C_3$–$C_9$)-alkyle($C_1$–$C_4$),
cycloalcényl($C_5$–$C_9$)-alkyle($C_1$–$C_4$),
un radical aryle éventuellement partiellement hydrogéné, ayant de 6 à 12 atomes de carbone, qui peut être substitué comme décrit plus haut pour R,
un radical aryl ($C_6$–$C_{12}$)-alkyle($C_1$–$C_4$) ou aroyl($C_7$–$C_{13}$)-alkyle($C_1$ ou $C_2$), qui l'un et l'autre peuvent être substitués comme le radical aryle précédent,
un radical hétéroaryle mono- ou bicyclique, éventuellement partiellement hydrogéné, ayant 5–7 ou 8–10 atomes nucléaires, dont 1 ou 2 atomes nucléaires représente(nt) un ou des atomes de soufre ou d'oxygène et/ou de 1 à 4 atomes nucléaires représente(nt) un ou des atomes d'azote, qui peut être substitué comme le radical aryle précédent,
ou la chaîne latérale éventuellement protégée d'un α-aminoacide $R^1$–$CH(NH_2)$–$COOH$ existant dans la nature,

$R^2$ et $R^3$ sont identiques ou différents, et représentent un atome d'hydrogène,
un groupe alkyle ayant de 1 à 6 atomes de carbone,
alcényle ayant de 2 à 6 atomes de carbone,
dialkyl($C_1$–$C_4$)-amino-alkyle($C_1$–$C_4$),
alcanoyloxy($C_1$–$C_5$)-alkyle($C_1$–$C_4$),
alcoxy($C_1$–$C_6$)-carbonyloxy-alkyle($C_1$–$C_4$),
aroyloxy($C_7$–$C_{13}$)-alkyle($C_1$–$C_4$),
aryloxy($C_6$–$C_{12}$).carbonyloxy-alkyle($C_1$–$C_4$),
aryle ayant de 6 à 12 atomes de carbone,
aryl($C_6$–$C_{12}$)-alkyle($C_1$–$C_4$),
cycloalkyle en $C_3$–$C_9$ ou
cycloalkyl($C_3$–$C_9$)-alkyle($C_1$–$C_4$), et

$R^4$ et $R^5$ ont les significations données plus haut, des fonctions amino, alkylamino, hydroxy, carboxy, mercapto et/ou guanidino libres présentes sur les radicaux R à $R^5$ étant éventuellement bloquées d'une façon connue en soi, pendant la réaction.

4. Procédé selon l'une des revendications 1 à 3, caractérisé en ce que l'on opère en présence d'anhydride n-propanephosphonique.

5. Procédé selon l'une des revendications 1 à 4, caractérisé en ce que l'on effectue la réaction dans un solvant organique inerte, en présence d'une amine organique tertiaire.

6. Procédé selon l'une des revendications 1 à 5, caractérisé en ce que l'on prépare le
[S,S,S,S,S]-N-[(1-carbéthoxy-3-phényl-propyl)-alanyl]-octahydroindole-2-carboxylate
de benzyle.

7. Procédé selon l'une des revendications 1 à 5, caractérisé en ce que l'on prépare le
N-[(1-(S)-carbéthoxy-3-phényl-propyl)-(S)-alanyl]-3aR,7aS-octahydroindole-2-(S)-carboxylate
de benzyle.

8. Procédé selon l'une des revendications 1 à 5, caractérisé en ce que l'on prépare le [S,S,S,S,S]-N-[(1-carbéthoxy-3-phényl-propyl)-alanyl]-décahydroisoquinoléine-3-carboxylate de benzyle.

9. Procédé selon l'une des revendications 1 à 5, caractérisé en ce que l'on prépare le [S,S,S]-N-[(1-carbéthoxy-3-phényl-propyl)-alanyl]-tétrahydroisoquinoléine-3-carboxylate de benzyle.

10. Procédé selon l'une des revendications 1 à 5, caractérisé en ce que l'on prépare le [S,S,S,S,S]-N-(1-carbéthoxy-3-phényl-propyl)-alanyl-2-azabicyclo[3.3.0]octane-3-carboxylate de benzyle.

11. Procédé selon l'une des revendications 1 à 5, caractérisé en ce que l'on prépare le N-(1-S-carbéthoxy-3-phényl-propyl)-S-alanyl-cis-endo-2-azabicyclo[3.1.0]hexane-3-S-carboxylate de benzyle.

12. Procédé selon l'une des revendications 1 à 5, caractérisé en ce que l'on prépare le N-(1-S-carbéthoxy-3-phényl-propyl)-S-alanyl-cis-endo-2,3,3a,4,5,7a-hexahydroindole-2-S-carboxylate de benzyle.

13. Procédé selon l'une des revendications 6 à 12, caractérisé en ce que, au lieu des esters benzyliques, on prépare les esters tert-butyliques correspondants ou d'autres esters dissociables par hydrogénolyse acide ou basique.